# DEMANDE DE BREVET EUROPEEN

(11) **EP 1 543 815 A1**
(43) Date de publication de la demande: **22.06.2005**
(21) Numéro de dépôt: 04292661.8
(22) Date de dépôt: 10.11.2004
(51) Int. Cl.: A61K 7/075

(54) **Composition cosmétique comprenant un agent cationique, un polymère semicristallin et une huile et procédé de traitement cosmétique**

(30) Priorité: 19.12.2003 FR 0351144
(71) Demandeur: L'OREAL, 75008 Paris (FR)
(72) Inventeur: Parris, Eric, 92110 Clichy (FR)
(74) Mandataire: Le Blainvaux, Françoise

(57) **Abrégé**

La présente invention est relative à une composition cosmétique comprenant, dans un milieu cosmétiquement acceptable, au moins au moins un agent cationique, au moins une huile et au moins un polymère semi-cristallin ayant un point de fusion supérieur ou égal à 30°C et à un procédé de traitement cosmétique des matières kératiniques en particulier les cheveux.

Ces compositions possèdent un effet conditionneur amélioré et rémanent.

## Description

La présente invention est relative à une composition cosmétique notamment de conditionnement des cheveux comprenant au moins un agent cationique, au moins un polymère semi-cristallin associé à au moins une huile et à un procédé de traitement cosmétique des matières kératiniques en particulier les cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.
Dans le domaine de la cosmétique, on cherche notamment à améliorer le conditionnement des cheveux. Par conditionnement, on entend des propriétés de démêlage facile, de brillance, de douceur au toucher et de glissant.

On a déjà proposé pour le traitement des matières kératiniques et en particulier des cheveux, des compositions cosmétiques contenant des tensioactifs cationiques.

De telles compositions présentent cependant des inconvénients tels que des problèmes de rinçabilité, des problèmes de stabilité, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques, de silicones cationiques ou de tensioactifs cationiques pour faciliter le démêlage des cheveux et pour leur communiquer douceur et souplesse. L'usage des polymères cationiques ou des cations dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

En résumé, il s'avère que les compositions cosmétiques actuelles de conditionnement, ne donnent pas complètement satisfaction. Ainsi, on cherche à obtenir des compositions cosmétiques ayant des propriétés de conditionnement améliorées en particulier un toucher plus lisse.

L'utilisation de polymère semi-cristallin est connue en cosmétique, et plus particulièrement dans le domaine du maquillage comme cela est décrit dans les demandes de brevet FR2824267. Des compositions cosmétiques dont la phase grasse est gélifiée par des polymères semi-cristallins ont été décrites la demande FR 2824264 qui décrit plus particulièrement des compositions solides de rouge à lèvre sous la forme d'un stick.

Les brevets US 5 736 125 et US 5 156 911, ainsi que la demande WO 01/19333 illustrent certains types de polymères semi-cristallins qui peuvent entrer dans la composition des formules de l'invention. Néanmoins, ces documents ne décrivent pas de compositions cosmétiques contenant un tensioactif cationique.

La demanderesse a maintenant découvert que l'association d'au moins un agent cationique, d'au moins un polymère semi-cristallin et d'au moins une huile, notamment dans des milieux non détergents ayant une concentration faible ( notamment inférieure à 2% en poids par rapport au poids total de la composition) ou nulle en agents tensio-actifs lavants permet de remédier à ces inconvénients.

Les cheveux traités avec cette composition sont lisses, se démêlent facilement sont brillants, souples, individualisés et ont un toucher doux et sans résidus. Les cheveux ont un aspect naturel et non chargé.

Sans être tenu par une quelconque théorie, il semblerait que dans ces conditions, on augmente significativement le dépôt d'huile sur les cheveux, d'où une efficacité accrue. Cette amélioration se fait cependant sans avoir un toucher chargé gras, ce qui est le cas habituellement lorsqu'on augmente la quantité d'huile.

Par ailleurs, cet effet de conditionnement peut être rémanent au rinçage.

Ainsi, selon la présente invention, il est maintenant proposé de nouvelles compositions cosmétiques notamment non lavantes , comprenant, dans un milieu cosmétiquement acceptable, au moins un agent cationique, au moins une huile et au moins un polymère semi-cristallin ayant un point de fusion supérieur ou égal à 30°C.

Un autre objet de l'invention consiste en un procédé de traitement cosmétique des matières kératiniques, en particulier les cheveux mettant en oeuvre la composition susvisée.

L'invention a encore pour objet l'utilisation de ladite composition comme après-shampoing.

D'autres objets, caractéristiques, aspects et avantages de l'invention apparaîtront encore plus clairement à la lecture de la description et des divers exemples qui suivent.

Selon un mode particulier de l'invention, l'huile est, de préférence, pré-épaissie par le polymère semi-cristallin, c'est à dire qu'on mélange l'huile et le polymère semi-cristallin avant l'introduction dans la composition.

Le rapport pondéral huile(s)/polymère(s) de l'invention est de préférence supérieur ou égal à 50/50, mieux encore supérieur ou égal à 60/40, plus préférentiellement compris entre 60/40 et 99/1 et encore plus préférentiellement compris entre 80/20 et 99/1.

Cette huile de préférence pré-épaissie est dispersée sous forme de particules dans la composition aqueuse. Les particules d'huile présentent de préférence une taille primaire moyenne en nombre comprise entre 1 et 100 µm, mieux encore entre 5 et 30 µm, et plus particulièrement de 10 à 20 µm .

Au sens de la présente invention, on entend par "taille primaire de particule", la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Par "polymère semi-cristallin", on entend au sens de l'invention, des polymères comportant une partie cristallisable et une partie amorphe dans le squelette et présentant une température de changement de phase réversible du premier ordre, en particulier de fusion (transition solide-liquide). La partie cristallisable est soit une chaîne latérale (ou chaîne pendante), soit une séquence dans le squelette.

Par "polymères", on entend au sens de l'invention des composés comportant au moins 2 motifs de répétition, de préférence au moins 3 motifs de répétition et plus spécialement au moins 10 motifs répétitifs.

Lorsque la partie cristallisable est une séquence du squelette polymérique, cette séquence cristallisable est de nature chimique différente de celle des séquences amorphes; le polymère semi-cristallin est dans ce cas un copolymère séquencé par exemple du type dibloc, tribloc ou multibloc. Lorsque la partie cristallisable est une chaîne pendante au squelette, le polymère semi cristallin peut être un homopolymère ou un copolymère.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.

Par "composé organique" ou "à structure organique", on entend des composés contenant des atomes de carbone et des atomes d'hydrogène et éventuellement des hétéroatomes comme S, O, N, P seuls ou en association.

### Les polymères semi-cristallins

De façon avantageuse, le ou les polymères semi-cristallins de la composition de l'invention ont une masse moléculaire moyenne en poids Mp supérieure ou égale à 1000, allant notamment de 5 000 à 1 000 000, de préférence de 10 000 à 500 000, préférentiellement de 15 000 à 500 000.

De préférence, le polymère semi-cristallin comporte i) un squelette polymérique et ii) au moins une chaîne latérale cristallisable et/ou une séquence organique cristallisable faisant partie du squelette dudit polymère semi-cristallin.

En particulier, le polymère semi-cristallin est choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

Le ou les polymères semi-cristallins selon l'invention servant d'agent structurant sont des solides à température ambiante (25°C) et pression atmosphérique (760 mm de Hg), dont la température de fusion est supérieure ou égale à 30°C, de préférence allant de 30 °C à 80 °C), et plus préférentiellement allant de 30 °C à 70 °C. Cette température de fusion est une température de changement d'état du premier ordre. Cette température de fusion peut être mesurée par toute méthode connue et en particulier à l'aide d'un calorimètre à balayage différentiel (D.S.C).
Les valeurs de point de fusion correspondent notamment au point de fusion mesuré à l'aide d'un calorimètre à balayage différentiel (D. S. C), tel que le calorimètre vendu sous la dénomination DSC 30 par la société METTLER, avec une montée en température de 5 ou 10°C par minute. (Le point de fusion considéré est le point correspondant à la température du pic le plus endotherme du thermogramme).

Le ou les polymères semi-cristallins selon l'invention ont de préférence une température de fusion supérieure à la température du support kératinique destiné à recevoir ladite composition, en particulier les cheveux.

Le ou les polymères semi-cristallins selon l'invention sont capables de structurer (d'épaissir) seuls ou en mélange, l'huile sans ajout de tensioactif particulier, ni de sel.

Selon l'invention les polymères semi-cristallins sont avantageusement solubles dans la phase grasse, notamment à au moins 1 % en poids, à une température supérieure à leur température de fusion. En dehors des chaînes ou séquences cristallisables, les séquences des polymères sont amorphes.

De préférence, le squelette polymérique des polymères semi-cristallins est soluble dans la phase grasse liquide.

De préférence, les séquences ou chaînes cristallisables des polymères semi-cristallins représentent au moins 30 % du poids total de chaque polymère et mieux au moins 40 %. Les polymères semi-cristallins à chaînes latérales cristallisables sont des homo ou des copolymères. Les polymères semi-cristallins de l'invention à séquences cristallisables sont des copolymères, séquencés ou multiséquencés. Ils peuvent être obtenus par polymérisation de monomère(s) à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, de préférence ils ne comportent pas de squelette polysaccharidique.

Les polymères semi-cristallins utilisables dans l'invention sont en particulier :
- les copolymères séquencés de polyoléfines à cristallisation contrôlée, dont les monomères sont décrits dans EP-A-0 951 897.
- les polycondensats et notamment de type polyester, aliphatique ou aromatique ou aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s), tels que décrits dans le document WO-A-01/19333,
- et leurs mélanges. Dans ces deux derniers cas, la ou les chaînes latérales ou séquences cristallisables sont hydrophobes.

### A) Polymères semi-cristallins à chaînes latérales cristallisables

On peut citer en particulier ceux définis dans les documents US-A-5 156 911 et WO-A-01/19333.
. Ce sont des homopolymères ou copolymères comportant de 50 à 100 % en poids de motifs résultant de la polymérisation d'un ou plusieurs monomères porteurs de chaîne latérale hydrophobe cristallisable.
. Ces homo- ou co-polymères sont de toute nature du moment qu'ils présentent les conditions indiquées ci-après avec en particulier la caractéristique d'être solubles ou dispersables dans la phase grasse liquide, par chauffage au-dessus de leur température de fusion Pf. Ils peuvent résulter :
   - de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
   - de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

a) D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule (I) : 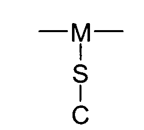 avec M représentant un atome du squelette polymérique
   S représentant un espaceur
   C représentant un groupe cristallisable
Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O)ₙ, linéaire ou ramifié ou cyclique, avec n entier allant de 0 à 22. De préférence « S » est un groupe linéaire. De préférence, « S » et « C » sont différents.

Lorsque les chaînes cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en C₁₄-C₂₄. de préférence en C₁₆-C₂₂. Lorsqu'il s'agit de chaînes alkyle fluorées ou perfluorées, elles comportent au moins 11 atomes de carbone dont au moins 6 atomes de carbone sont fluorés.

Comme exemple d'homopolymères ou de copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome(s) de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :
α) de Y qui est un monomère polaire ou non polaire ou un mélange des deux :
   . Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   . Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en C₁ à C₁₀, comme l'α-méthylstyrène, un macromonomère du type polyorganosiloxane à insaturation vinylique.

   Par "alkyle", on entend au sens au sens de l'invention un groupement saturé notamment en C₈ à C₂₄, sauf mention exprès.
β) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

### B) Les polymères portant dans le squelette au moins une séquence cristallisable

Il s'agit encore de polymères solubles ou dispersables dans la phase grasse liquide par chauffage au-dessus de leur point de fusion Pf. Ces polymères sont notamment des copolymères séquencés constitués d'au moins deux séquences de nature chimique différente dont l'une est cristallisable.
- On peut utiliser les polymères définis dans le brevet US-A-5 156 911,
- Les copolymères séquencés d'oléfine ou de cyclooléfine à chaîne cristallisable comme ceux issus de la polymérisation séquencée de :
   . cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
   . avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
   . et en particulier les copoly(éthylène/norbornène) blocs et les terpolymères (éthylène/propylène/éthylidène-norbornène), blocs. On peut aussi utiliser ceux résultants de la copolymérisation séquencée d'au moins 2 α-oléfines en C₂-C₁₆ et mieux en C₂-C₁₂ tels que ceux cités précédemment et en particulier les bipolymères séquencés d'éthylène et d'1-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   . Séquence cristallisable par nature : a) polyester comme les poly(alkylène téréphtalate), b) polyoléfine comme les polyéthylènes ou polypropylènes.
   . Séquence amorphe et lipophile comme: les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
α) les copolymères séquencés poly(e-caprolactone)-b-poly(butadiène), utilisés de préférence hydrogénés, tels que ceux décrits dans l'article "Melting behavior of poly(e-caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et "Polymer agregates with crystalline cores : the system poly(ethylene)-poly(ethylene-propylene)" P. Richter et al., Macromolécules, 30, 1053-1068 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés à partir du moment où le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N alkyl (méth)acrylamides en C₁₄ à C₂₄ avec ou sans atome(s) de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alphaoléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré, qui peut être représenté par la formule suivante : 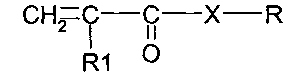 dans laquelle R₁ est H ou CH₃, R représente un groupe alkyle en C1-C10 éventuellement fluoré et X représente O, NH ou NR₂, où R₂ représente un groupe alkyle en C₁-C₁₀ éventuellement fluoré.

A titre d'exemple particulier de polymère semi-cristallins structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule (I) précédente.

Dans la suite de la description, le ou les polymères semi-cristallins ayant une température de fusion Pf₂ inférieure à 50°C seront dénommés "polymères à bas point de fusion" et le ou les composés cristallins ou semi-cristallins ayant une température de fusion Pf₁ supérieure ou égale à 50°C seront dénommés "composés à haut point de fusion". Selon l'invention, le point de fusion peut être mesuré notamment par toute méthode connue et en particulier avec un calorimètre à balayage différentiel (D.S.C).

Selon l'invention le ou les composés semi-cristallins à haut point de fusion sont avantageusement des polymères ayant une température de fusion Pf₁ telle que 50°C ≤ Pf₁ ≤ 150°C, mieux 55°C ≤ Pf₁ ≤ 150°C, et de préférence 60°C ≤ Pf₁ ≤ 130°C et les polymères à bas point de fusion ont avantageusement une température de fusion Pf₂ telle que 30°C ≤ Pf₂ < 50°C, et mieux 35°C ≤ Pf₂ ≤ 45°C. Cette température de fusion est une température de changement d'état du premier ordre.

De façon générale, les polymères à bas point de fusion présentent une température de fusion Pf₂ au moins égale à la température du support kératinique devant recevoir la composition selon l'invention.

Comme polymère semi-cristallin à haut point de fusion utilisable dans l'invention, on peut citer des polymères cristallins, solides à température ambiante et ayant une température de fusion supérieure à 50°C comme des polymères statistiques comportant une cristallisation contrôlée, tels que décrits dans le document EP-A-0 951 897 et plus spécialement les produits commerciaux Engage 8 401 et Engage 8 402 de Dupont de Nemours, respectivement de température de fusion de 51°C et 64°C et qui sont des bipolymères statistiques éthylène/1-octène.
i) Les polymères semi-cristallins présentant un point de fusion supérieur ou égal à 50°C sont notamment l'Intelimer décrit dans la brochure "Intelimer® polymers", Landec IP22 (Rev. 4-97) de température de fusion de 56°C, qui est un produit visqueux à température ambiante, imperméable, non-collant.
   On peut aussi utiliser les polymères semi-cristallins obtenus par copolymérisation de l'acrylate de béhényle et de l'acide acrylique ou de NVP, tels que décrits dans les documents US-A-5 519 063 et EP-A- 055 0745 et plus spécialement ceux décrits dans les exemples 3 et 4, ci-après, de préparation de polymère.
ii) Les polymères semi-cristallins dont le point de fusion est inférieur à 50°C sont notamment ceux décrits dans les exemples 3, 4, 5, 7, 9 du brevet US-A-5 156 911 à groupement -COOH, résultant de la copolymérisation d'acide acrylique et d'alkyl(méth)acrylate en C₅ à C₁₆ (de température de fusion allant de 20°C à 35°C) et plus particulièrement résultant de la copolymérisation :
   . d'acide acrylique, d'hexadécylacrylate et d'isodécylacrylate dans un rapport 1/16/3,
   . d'acide acrylique et de pentadécylacrylate dans un rapport 1/19,
   . d'acide acrylique, d'hexadécylacrylate, éthylacrylate dans un rapport 2,5/76,5/20,
   . d'acide acrylique, d'hexadécylacrylate et de méthylacrylate dans un rapport 5/85/10,
   . d'acide acrylique, d'octadécylméthacrylate dans un rapport 2,5/97,5,
   d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le polymère Structure « O » de National Starch tel que celui décrit dans le document US-A-5 736 125 de température de fusion de 44°C ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins de bas point de fusion obtenus par copolymérisation d'acrylate de stéaryle et d'acide acrylique ou de NVP tels que décrits dans le document US-A-5519063 ou EP-A-550745 et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745.

De préférence, les polymères semi-cristallins à bas point de fusion et/ou ceux à haut point de fusion ne comportent pas de groupement carboxylique.

Selon un mode plus particulier de réalisation de l'invention, le polymère est issu d'un monomère à chaîne cristallisable choisi parmi les (méth)acrylates d'alkyle saturés en C₁₄ à C₂₂ et encore plus particulièrement les poly(acrylate de stéaryle) ou les poly(acrylate de béhényle).

L'épaississement de la phase grasse est modulable selon la nature du ou des polymères et leurs concentrations et peut être telle que l'on obtienne de préférence une viscosité allant de 1000 à 250.000 cps et de préférence de 10.000 à 50.000 cps. Mesurée à 25°C avec un appareil RHEOMAT 180 avec un taux de cisaillement de 100s⁻¹.

Le polymère semi-cristallin tel que défini ci-dessus est de préférence présent en une quantité comprise entre 0,005 et 20 % en poids, mieux encore en une quantité comprise entre 0,01 et 10 % en poids, et encore plus préférentiellement en une quantité comprise entre 0,01 et 1% en poids par rapport au poids total de la composition.

Par huile, au sens de la demande, on entend un corps gras liquide insoluble dans l'eau à température ambiante (25°C) et pression atmosphérique (760 mm de Hg). La phase huileuse peut être composée d'une ou plusieurs huiles, compatibles entre elles.

Par insoluble dans l'eau, on entend au sens de la présente invention, une substance qui présente une solubilité dan l'eau pure inférieure à 1 % à 25 °C et sous pression atmosphérique.

Les huiles utilisées dans la présente invention présente une viscosité dynamique à 25 °C, inférieure à 1 Pa.s (1000 cps), de préférence comprise entre 10⁻³ et 0,1 Pa.s (1 et 100 cps). La viscosité dynamique est mesurée à 25 °C avec un taux de cisaillement de 100 s⁻¹, par exemple, avec l'appareil référencé RM 180 Rheomat de la société METTLER.

Les huiles pouvant être utilisées dans la présente invention sont choisies de préférence, parmi les huiles végétales, les huiles minérales, les huiles de synthèse, et les esters d'acide gras.

Parmi les huiles végétales pouvant être utilisées dans la présente invention, on peut notamment citer l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot et l'huile de calophyllum.

Des exemples d'huiles minérales sont l'huile de paraffine et l'huile de vaseline. Les huiles de synthèse peuvent notamment être choisies parmi les polydécènes, le squalane, les poly(alpha-oléfines) comme l'isododécane ou l'isohexadécane, les huiles végétales transestérifiées et les huiles fluorées.

On peut également utiliser des esters d'acide gras, tels que par exemple, les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle.

Les huiles particulièrement préférées dans le cadre de la présente invention sont l'huile d'avocat, l'huile de ricin, l'huile d'olive, le polydécène hydrogéné, le myristate d'isopropyle, l'isononanoate d'isononyle, une paraffine liquide

La ou les huile(s) telle(s) que définie(s) ci-dessus est ou sont de préférence présente(s) en une quantité comprise entre 0,01 et 30 % en poids, encore plus préférentiellement en une quantité comprise entre 0,1 et 15 % en poids et plus particulièrement de 0,1 à 10% en poids par rapport au poids de la composition et notamment de 0,1 à 5% en poids.

Les agents cationiques sont de préférence choisis parmi les tensioactifs cationiques et les polymères cationiques et/ou leur mélanges et plus particulièrement parmi les tensioactifs cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 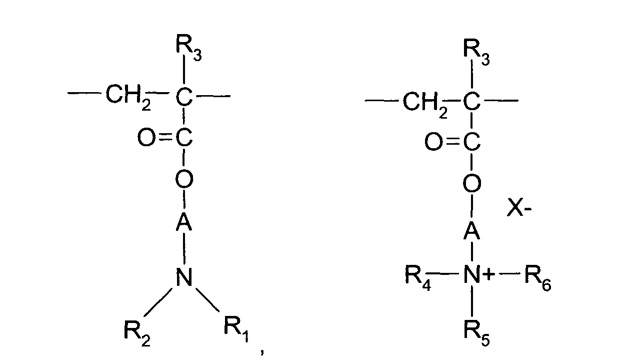 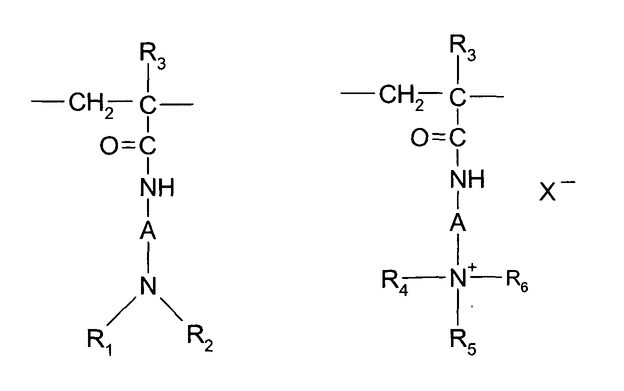 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires sont notamment décrits dans le brevet français 1 492 597, et sont en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyoxyléthyl triméthylammonium, de méthacrylamidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (II) ou (III) : 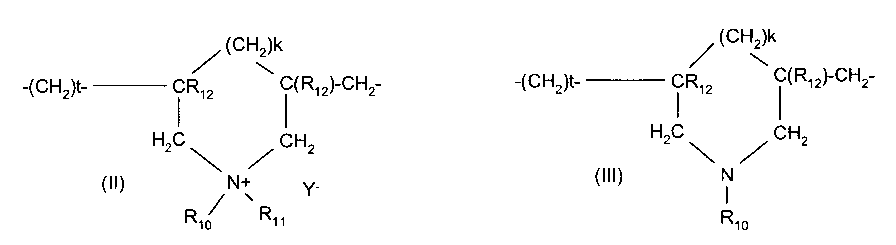 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide commercialisés sous la dénomination "MERQUAT 550".
(8) les polymères de diammonium quaternaire comprenant des motifs récurrents répondant à la formule (IV): 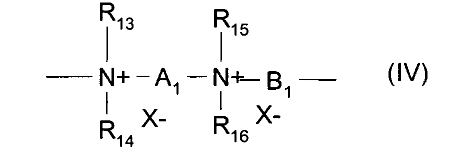 formule (IV) dans laquelle :
   R₁₃, R₁₄, R₁₅ et R₁₆, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;
   n, identique ou différent, représente un nombre entier allant de 2 à 20, de préférence de 2 à 6,
   De préférence, X⁻ est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (V): 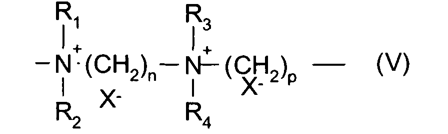 dans laquelle R₁, R₂, R₃ et R_{4,} identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (V) particulièrement préféré est celui pour lequel R₁, R₂, R₃ et R₄, représentent un radical méthyle et n = 3, p = 6 et X = Cl, dénommé Hexadimethrine chloride selon la nomenclature lNCl (CTFA).
(9)les polymères de polyammonium quaternaires comprenant des motifs de formule (VI): 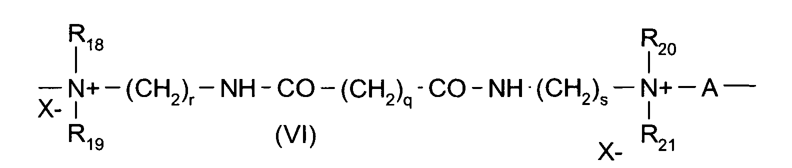 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol® A 15", "Mirapol® AD1 ", "Mirapol® AZ1" et "Mirapol® 175" vendus par la société Miranol.
(10)Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.
(12) Les polymères de préférence réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination « JR 400 » par la Société AMERCHOL, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,01 à 20%, de préférence de 0,05 à 10% et plus particulièrement de 0,1 à 5 % en poids par rapport au poids total de la composition.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaire, on peut notamment citer, par exemple :
- ceux qui présentent la formule générale (V) suivante : 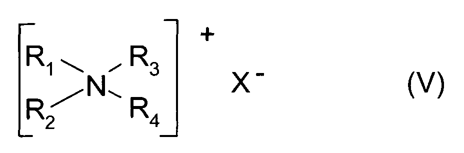 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
   - les sels d'ammonium quaternaire de l'imidazoline comme, par exemple, ceux de formule (VI) suivante : 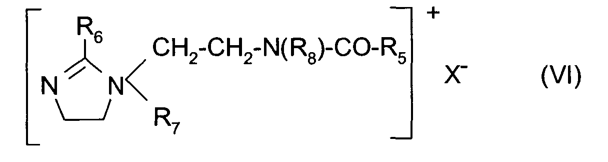 dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,
   - les sels de diammonium quaternaire de formule (VII) : 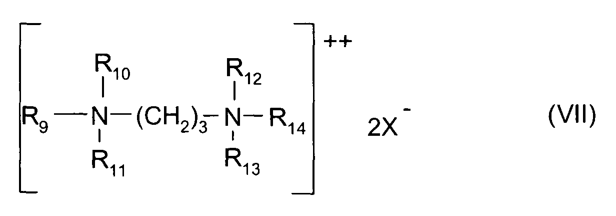 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
   - les sels d'ammonium quaternaire contenant au moins une fonction ester, tels que ceux de formule (VIII) suivante : 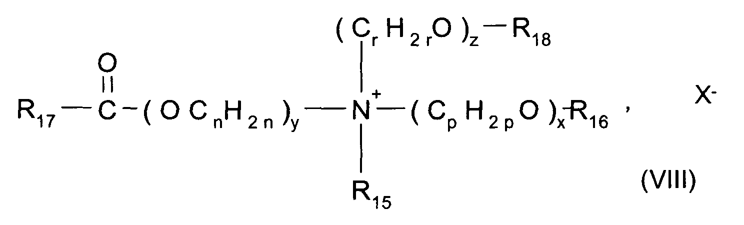 dans laquelle :
      R₁₅ est choisi parmi les radicaux alkyles en C₁-C₆ et les radicaux hydroxyalkyles ou dihydroxyalkyles en C₁-C₆ ;
      R₁₆ est choisi parmi :
         - le radical 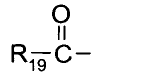
         - les radicaux R₂₀ hydrocarbonés en C₁-C₂₂, linéaires ou ramifiés, saturés ou insaturés,
         - l'atome d'hydrogène,
      R₁₇ est choisi parmi :
         - le radical 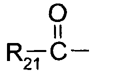
         - les radicaux R₂₂ hydrocarbonés en C₁-C₆, linéaires ou ramifiés, saturés ou insaturés,
         - l'atome d'hydrogène,
      R₁₈, R₁₉ et R₂₁, identiques ou différents, sont choisis parmi les radicaux hydrocarbonés en C₇-C₂₁, linéaires ou ramifiés, saturés ou insaturés ;
      r, n et p, identiques ou différents, sont des entiers valant de 2 à 6 ;
      y est un entier valant de 1 à 10 ;
      x et z, identiques ou différents, sont des entiers valant de 0 à 10 ;
      X⁻ est un anion simple ou complexe, organique ou inorganique ;
   sous réserve que la somme x + y + z vaut de 1 à 15, que lorsque x vaut 0 alors R₁₆ désigne R₂₀ et que lorsque z vaut 0 alors R₁₈ désigne R₂₂.
   Les radicaux alkyles R₁₅ peuvent être linéaires ou ramifiés, et plus particulièrement linéaires.
   De préférence, R₁₅ désigne un radical méthyle, éthyle, hydroxyéthyle ou dihydroxypropyle, et plus particulièrement un radical méthyle ou éthyle.
   Avantageusement, la somme x + y + z vaut de 1 à 10.
   Lorsque R₁₆ est un radical R₂₀ hydrocarboné, il peut être long et avoir de 12 à 22 atomes de carbone, ou court et avoir de 1 à 3 atomes de carbone.
   Lorsque R₁₈ est un radical R₂₂ hydrocarboné, il a de préférence 1 à 3 atomes de carbone.
   Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.
   De préférence, x et z, identiques ou
   différents, valent 0 ou 1.
   Avantageusement, y est égal à 1.
   De préférence, r, n et p, identiques ou différents, valent 2 ou 3, et encore plus particulièrement sont égaux à 2.
   L'anion X⁻ est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
   L'anion X⁻ est encore plus particulièrement le chlorure ou le méthylsulfate.
   On utilise plus particulièrement dans la composition selon l'invention, les sels d'ammonium de formule (IV) dans laquelle :
   - R₁₅ désigne un radical méthyle ou éthyle,
   - x et y sont égaux à 1 ;
   - z est égal à 0 ou 1 ;
   - r, n et pt sont égaux à 2 ;
   - R₁₆ est choisi parmi:
   - le radical 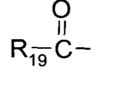
   - les radicaux méthyle, éthyle ou hydrocarbonés en C₁₄-C₂₂,
   - l'atome d'hydrogène ;
   - R₁₈ est choisi parmi :

   - le radical 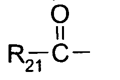
   - l'atome d'hydrogène ;
   - R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.
   Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.
Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéarylamidopropyldiméthyl-(myristyl acétate)-ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

La composition selon l'invention contient de préférence le ou les tensioactifs cationiques en une quantité allant de 0,05 à 10 % en poids, de préférence de 0,1 à 8 % en poids par rapport au poids total de la composition et plus particulièrement de 0,2 à 6% en poids et encore plus préférentiellement de 0,3 à 3% en poids.

La composition selon l'invention peut contenir éventuellement d'autres tensioactifs que les tensioactifs cationiques, notamment non ioniques ou amphotères.

Les tensioactifs additionnels sont généralement présents une quantité comprise entre 0,1% et 10% en poids environ, de préférence entre 0,5% et 8% et encore plus préférentiellement entre 1% et 5%, par rapport au poids total de la composition.

La composition selon l'invention contient de préférence au moins un tensioactif choisi parmi les tensioactifs non ioniques.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés, ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

Les compositions non lavantes (non détergentes) comprennent de préférence moins de 4% en poids de tensioactifs détergents, anioniques et plus particulièrement moins de 1% en poids par rapport au poids total de la composition.

La composition selon l'invention peut comprendre en outre au moins un agent conditionneur.

Les agents conditionneurs en particulier les polymères cationiques sont contenus de préférence dans la composition selon l'invention en une quantité allant de 0,01 % à 20 % en poids, mieux encore allant de 0,05 % à 10 % en poids et plus particulièrement allant de 0,1 % à 5 % en poids par rapport au poids total de la composition.

Le milieu cosmétiquement acceptable est de préférence aqueux et peut comprendre de l'eau ou un mélange d'eau et d'un solvant cosmétiquement acceptable tel qu'un alcool inférieur en C₁-C₄, par exemple l'éthanol, l'isopropanol, le tertio-butanol, le n-butanol ; les polyols comme le propylèneglycol, la glycérine ; les éthers de polyols ; les alcanes en C₅-C₁₀ ; et leurs mélanges.. Les solvants sont de préférence choisis parmi la glycérine et le propylène glycol.

Le milieu cosmétiquement acceptable notamment aqueux représente de 30 à 98% en poids par rapport au poids total de la composition et de préférence de 80 à 98% en poids.

Les solvants sont de préférence présents dans des concentrations allant de 0,5 à 30% en poids par rapport au poids total de la composition.

Le pH des compositions de l'invention est compris entre 2 et 8, de préférence entre 3 et 7,5.

Les compositions selon l'invention peuvent également contenir des additifs classiques bien connus dans la technique, choisis parmi les filtres solaires siliconés ou non, les tensioactifs cationiques, les protéines, les hydrolysats de protéines, les céramides, les pseudocéramides, les cires telles que les cires végétales, les acides gras à chaînes linéaires ou ramifiées en C₁₂-C₄₀ tels que l'acide méthyl-18 eicosanoique, les hydroxyacides, les vitamines telles que notamment les vitamines E, C, B, les provitamines tels que le panthénol, les silicones, les huiles animales, minérales ou de synthèse, les polymères anioniques, cationiques, non-ioniques amphotères ou zwitteroniques différents des polymères semi-cristallins selon l'invention, les filtres UV, les parfums, les colorants, les épaississants polymériques naturels ou synthétiques, des épaississants non polymériques comme des acides ou des électrolytes,les alcools gras en C₁₂-C₃₀, les agents nacrants, les conservateurs, les agents de stabilisation de pH, les agents antimicrobiens, les agents anti-pelliculaires ou anti-séborrhéiques, les agents anti-oxydants ou réducteurs, les agents acides ou alcalins et leurs mélanges et tout autre additif classiquement utilisé dans le domaine cosmétique qui n'affecte pas les propriétés des composition selon l'invention.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de l'invention peuvent se présenter sous forme d'après-shampooing à rincer ou non, de compositions pour permanente, défrisage, coloration ou décoloration, ou encore sous forme de compositions éventuellement à rincer, à appliquer avant ou après une coloration, une décoloration, une permanente ou un défrisage ou encore entre les deux étapes d'une permanente ou d'un défrisage.
Elles peuvent être utilisées, par exemple, comme après-shampoings, soins, masques de soin profond, lotions ou crèmes de traitement du cuir chevelu. Ces compositions peuvent être rincées ou non rincées.

Selon un mode de réalisation préféré de l'invention, la composition peut être utilisée comme après-shampoing, en particulier sur des cheveux fins. Cet après-shampooing peut être rincé ou non rincé et de préférence rincé.

Les compositions cosmétiques selon l'invention peuvent se présenter sous forme de gel, de lait, de crème, d'émulsion, de lotion fluides ou épaissies ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

La présente invention concerne également un procédé de traitement cosmétique des matières kératiniques telles que par exemple la peau ou les cheveux qui consiste à appliquer une quantité efficace d'une composition cosmétique telle que décrite ci-dessus, sur les matières kératiniques, à effectuer un éventuel rinçage après un éventuel temps de pose.
Le rinçage s'effectue par exemple avec de l'eau.

Ainsi, ce procédé selon l'invention permet le maintien de la coiffure, le traitement, le conditionnement, le soin des cheveux ou de toute autre matière kératinique.

Les exemples suivants illustrent la présente invention et ne doivent être considérés en aucune manière comme limitant l'invention.

### EXEMPLES 1 ET 2

On a préparé les compositions suivantes d'après-shampooing à rincer :

| Composition | 1 | 2 |
|---|---|---|
| Inuline (Raftiline HP- ORAFTI) | 15 g | --- |
| Polyquaternium-37 (Salcare SC 95-CIBA) | 0.5 g | --- |
| Alcool Cétylstéarylique | --- | 2 g |
| Cetyl esters (Spermwax vegetal-ROBECO) | --- | 0.25 g |
| Behentrimonium Chloride (Genamin KDMP - CLARIANT) | --- | 1.4 g |
| Mélange (6/94 en poids) de poly C10-30 alkyl acrylate (Intelimer IPA13-1 de LANDEC) et de Polydécène hydrogéné (CERAFLOW E de SHAMROCK) | 0.5 g | 1 g |
| Conservateur | qs | qs |
| Agent de pH qs | pH = 7 | pH = 3.5 |
| Eau | Qsp 100 g | Qsp 100 g |

On a appliqué ces compositions sur des cheveux sensibilisés. Après environ 3 minutes de pose, on rince les cheveux. Ces cheveux sont alors très lisses.

### EXEMPLES 3-4

On prépare les compositions suivantes d'après-shampooing à rincer :

| Composition | 3 | 4 |
|---|---|---|
| Inuline (Raftiline HP-ORAFTI) | 15 g | --- |
| Polyquaternium-37 (Salcare SC 95-CIBA) | 0.5 g | --- |
| Alcool Cétylstéarylique | --- | 2 g |
| Cétyl esters (Spermwax vegetal-ROBECO) | --- | 0.25 g |
| Behentrimonium Chloride (Genamin KDMP - CLARIANT) | --- | 1.4 g |
| Mélange (6/94 en poids) de poly C10-30 alkyl acrylate (Intelimer IPA13-1 de LANDEC) et d'huile d'avocat | 0.5 g | 1 g |
| Conservateur | qs | qs |
| Agent de pH qs | pH = 7 | pH = 3.5 |
| Eau | Qsp 100 g | Qsp 100 g |

On applique ces compositions sur des cheveux sensibilisés. Après environ 3 minutes de pose, on rince les cheveux. Ces cheveux sont alors très lisses.

### EXEMPLES 5-6

On prépare les compositions suivantes d'après-shampooing à rincer :

| Composition | 5 | 6 |
|---|---|---|
| Inuline (Raftiline HP- ORAFTI) | 15 g | --- |
| Polyquaternium-37 (Salcare SC 95-CIBA) | 0.5 g | --- |
| Alcool Cétylstéarylique | --- | 2 g |
| Cétyl esters (Spermwax vegetal-ROBECO) | --- | 0.25 g |
| Behentrimonium Chloride (Genamin KDMP - CLARIANT) | --- | 1.4 g |
| Mélange (15/85 en poids) de copolymère C10-30 alkyl acrylate/acide méthacrylique et d'isononanoate d'isononyle | 0.5 g | 1 g |
| Conservateur | qs | qs |
| Agent de pH qs | pH = 7 | pH = 3.5 |
| Eau | Qsp 100 g | Qsp 100 g |

On applique ces compositions sur des cheveux sensibilisés. Après environ 3 minutes de pose, on rince les cheveux. Ces cheveux sont alors très lisses.

## Revendications

1. Composition cosmétique, comprenant, dans un milieu cosmétiquement acceptable, au moins un agent cationique, au moins une huile et au moins un polymère semi-cristallin ayant un point de fusion supérieur ou égal à 30°C.

2. Composition selon la revendication 1, **caractérisée par le fait que** le polymère semi-cristallin a un point de fusion allant de 30 °C à 80 °C, de préférence allant de 30 °C à 70 °C.

3. Composition selon l'une des revendications précédentes, dans laquelle le polymère semi-cristallin présente une masse moléculaire moyenne en poids supérieur ou égale à 1 000.

4. Composition selon l'une des revendications précédentes, **caractérisée en ce que** le polymère semi-cristallin a une masse moléculaire moyenne en nombre allant de 5 000 à 1 000 000 et mieux de 10 000 à 500 000.

5. Composition selon l'une des revendications précédentes, dans laquelle le polymère semi-cristallin comporte i) un squelette polymérique et ii) au moins une chaîne latérale cristallisable et/ou une séquence organique cristallisable faisant partie du squelette dudit polymère semi-cristallin.

6. Composition selon l'une des revendications précédentes, **caractérisée en ce que** le polymère semi-cristallin est choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

7. Composition selon l'une des revendications précédentes, **caractérisée en ce que** la séquence cristallisable est de nature différente de la séquence amorphe.

8. Composition selon l'une quelconque des revendications précédentes, **caractérisée en ce que** le polymère semi-cristallin a une chaîne organique cristallisable et/ou une séquence cristallisable représentant au moins 30 % et de préférence 40% du poids total du polymère.

9. Composition selon l'une des revendications précédentes, **caractérisée en ce que** le polymère semi-cristallin est choisi parmi :
- les copolymères séquencés de polyoléfines à cristallisation contrôlée,
- les polycondensats polyesters aliphatiques ou aromatiques et les copolyesters aliphatiques/aromatiques,
- les homo- ou co-polymères portant au moins une chaîne latérale cristallisable,
- leurs mélanges.

10. Composition selon l'une des revendications précédentes, caractérisée en ce le polymère semi-cristallin est choisi parmi les homopolymères et copolymères comportant de 50 à 100 % en poids de motifs résultant de la polymérisation de un ou plusieurs monomères porteurs de de chaîne(s) latérale(s) hydrophobe(s) cristallisable(s).

11. Composition selon l'une des revendications précédentes, **caractérisée en ce que** le polymère semi-cristallin est choisi parmi les homopolymères et copolymères séquencés résultant de la polymérisation d'au moins un monomère à séquence(s) amorphe(s) ou chaîne(s) latérale(s) cristallisable(s) par motif de répétition , leur mélanges de formule (I) : 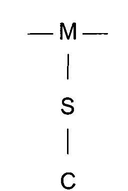 avec M représentant un atome du squelette polymérique, S représentant un espaceur, C représentant un groupe cristallisable et leurs mélanges, avec « S-C » représentant une chaîne alkyle à au moins 11 atomes de carbone, éventuellement fluorée ou perfluorée.

12. Composition selon l'une des revendications précédentes, **caractérisée en ce que** le polymère semi-cristallin est choisi parmi les polymères résultant de la polymérisation d'au moins un monomère choisi parmi l'acide acrylique, méthacrylique, crotonique, itaconique, maléique, l'anhydride maléique et leurs mélanges.

13. Composition selon l'une des revendications précédentes, **caractérisée en ce que** le polymère semi-cristallin est choisi parmi les homopolymères et copolymères résultant de la polymérisation d'au moins un monomère à séquence cristallisable choisis parmi les (méth)acrylates d'alkyle saturés en C₁₄-C₂₄, les (méth)acrylates de perfluoroalkyle en C₁₁-C₁₅, les N-alkyl (méth)acrylamides en C₁₄ à C₂₄ avec ou sans atome(s) de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, leurs mélanges.

14. Composition selon l'une quelconque des revendications précédentes, **caractérisée en ce que** le polymère semi-cristallin est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N-alkyl (méth)acrylamides en C₁₄ à C₂₄ avec ou sans atome(s) de fluor, les esters vinyliques à chaînes alkyle ou perfluoroalkyle en C₁₄ à C₂₄, les éthers vinyliques à chaîne alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré.

15. Composition selon l'une quelconque des revendications précédentes, **caractérisée par le fait que** le polymère semi-cristallin est choisi parmi les homopolymères d'alkyl(méth)acrylate ou d'alkyl(méth)acrylamide avec un groupe alkyle en C₁₄ à C₂₄, les copolymères de ces monomères avec un monomère hydrophile.

16. Composition selon l'une quelconque des revendications précédentes, caractérisée en par le fait que les polymères semi-cristallins sont des copolymères d'alkyl(méth)acrylate ou d'alkyl (méth)acrylamide avec un groupe alkyle en C₁₄ à C₂₄ avec un monomère hydrophile de nature différente de l'acide (méth)acrylique comme la N-vinylpyrolidone ou l'hydroxyéthyl (méth)acrylate, et leurs mélanges.

17. Composition selon l'une quelconque des revendications précédentes, **caractérisée en ce que** le polymère semi-cristallin est issu d'un monomère à chaîne cristallisable choisi parmi les (méth)acrylates d'alkyle saturés en C₁₄ à C₂₂.

18. Composition selon la revendication 17, **caractérisée en ce que** le polymère semi-cristallin est choisi parmi les poly(acrylate de stéaryle) ou les poly(acrylate de béhényle).

19. Composition selon l'une quelconque des revendications précédentes, **caractérisée par le fait que** les polymères de poids moléculaire élevé sont présents dans les composition de l'invention à une concentration comprise entre 0,01 et 10% en poids, de préférence entre 0,05 et 5% par rapport au poids total de la composition.

20. Composition selon l'une quelconque des revendications précédentes, **caractérisée en ce que** l'huile est choisie parmi les huiles végétales, les huiles minérales, les huiles de synthèse, et les esters d'acides gras.

21. Composition selon la revendication 20, **caractérisée en ce que** l'huile est choisie parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile de paraffine et l'huile de vaseline ; les polydécènes, le squalane, les poly(alpha-oléfines) comme l'isododécane ou l'isohexadécane, les huiles végétales transestérifiées et les huiles fluorées ; les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone.

22. Composition selon la revendication 21, **caractérisée en ce que** l'huile est choisie parmi l'huile d'avocat, l'huile de ricin, l'huile d'olive, l'isohexadécane, le polydécène , le myristate d'isopropyle, l'isononanoate d'isononyle, une paraffine liquide.

23. Composition selon l'une quelconque des revendications précédentes, **caractérisée en ce que** la ou le(s) huile(s) est ou sont présente(s) en une quantité comprise entre 0,01 et 30 % en poids, de préférence entre 0,1 et 15 % en poids par rapport au poids total de la composition.

24. Composition selon l'une quelconque des revendications précédentes, **caractérisée en ce que** l'huile est pré-épaissie par le polymère semi-cristallin.

25. Composition selon l'une quelconque des revendications précédentes, **caractérisée en ce que** le rapport pondéral huile(s)/polymère semi-cristallin est supérieur ou égal à 50/50.

26. Composition selon la revendication 25, **caractérisée en ce que** le rapport pondéral huile(s)/ polymère semi-cristallin est supérieur ou égal à 60/40, de préférence compris entre 60/40 et 99/1.

27. Composition selon l'une quelconque des revendications précédentes, **caractérisée en ce que** l'huile présente une taille primaire moyenne en nombre comprise entre 1 et 100 µm, de préférence comprise entre 5 et 30 µm.

28. Composition selon l'une quelconque des revendications précédentes, **caractérisée en ce que** l'agent cationique est choisi parmi les polymères cationiques et les tensioactifs cationiques.

29. Composition selon la revendication 28, **caractérisée en ce que** les polymères cationiques sont choisis parmi les dérivés d'éther de cellulose quaternaires, les cyclopolymères cationiques, en particulier les homopolymères ou copolymères de chlorure de diméthyldiallylammonium, les polymères quaternaires de vinylpyrrolidone et de vinylimidazole, les homopolymères ou copolymères, éventuellement réticulés, de sels de méthacryloyloxyalkyl(C₁-C₄) trialkyl(C₁-C₄)ammonium, et leurs mélanges.

30. Composition selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

31. Composition selon la revendication 30, **caractérisée en ce que** les sels d'ammonium quaternaire sont choisis parmi :
- ceux qui présentent la formule générale (V) suivante : 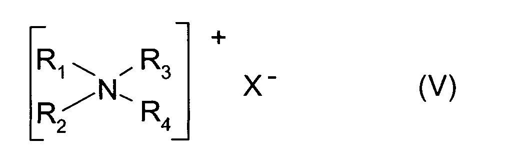
dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (VII) : 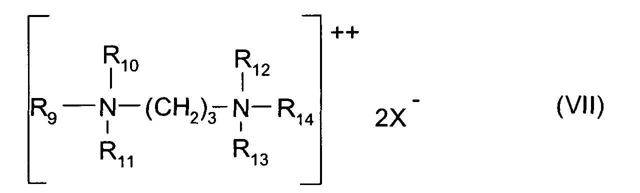 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates et méthylsulfates ;
- les sels d'ammonium quaternaire contenant au moins une fonction ester.

32. Composition selon la revendication 31, **caractérisée en ce que** les sels d'ammonium quaternaire de l'imidazoline sont choisis parmi ceux de formule (VI) suivante : 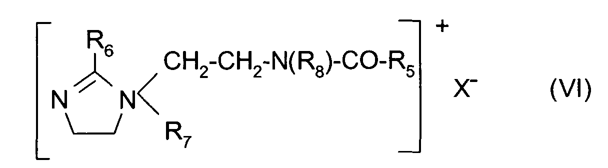 dans laquelle R₅ représente un radical alcényle ou alkyle comportant de 8 à 30 atomes de carbone par exemple dérivés des acides gras du suif ou de coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.

33. Composition selon l'une quelconque des revendications précédentes, **caractérisée en ce que** les tensioactifs cationiques sont choisis parmi le chlorure de béhényltriméthylammonium, le chlorure de cétyltriméthylammonium, le Quaternium-83, le chlorure de béhénylamidopropyl 2,3-dihydroxypropyl diméthyl ammonium et le chlorure de palmitylamidopropyltriméthylammonium.

34. Composition selon l'une quelconque des revendications précédentes, **caractérisée en ce que** les tensioactifs cationiques sont contenus en une quantité de 0,05 à 10 % en poids, de préférence de 0,1 à 8 % en poids par rapport au poids total de la composition.

35. Composition selon l'une quelconque des revendications précédentes, **caractérisée en ce qu'**elle comprend en outre au moins un agent conditionneur additionnel.

36. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

37. Composition selon la revendication 36, **caractérisée en ce que** le solvant cosmétiquement acceptable est choisi parmi les alcools inférieur en C₁-C₄ tel que l'éthanol, l'isopropanol, le tertio-butanol, le n-butanol ; les alkylèneglycols comme le propylèneglycol, les éthers de polyol ; les alcanes en C₅-C₁₀ et leurs mélanges.

38. Composition selon l'une quelconque des revendications précédentes, **caractérisée en ce qu'**elle comprend en outre des additifs tels que des agents tensioactifs cationiques, des polymères anioniques, cationiques, non-ioniques amphotères ou zwitteroniques, des filtres UV, des parfums, des colorants, des épaississants naturels ou synthétiques, des alcools gras en C₁₂-C₃₀, des agents nacrants, des conservateurs, des agents de stabilisation de pH, des vitamines, des provitamines, des agents antimicrobiens, des agents anti-pélliculaires ou anti-séborrhéiques, les agents anti-oxydants ou réducteurs, et les agents acides ou alcalins et leurs mélanges.

39. Composition selon l'une quelconque des revendications précédentes, **caractérisée par le fait qu'**elle se présente sous forme d'après-shampooing à rincer ou non, de composition pour la permanente, le défrisage, la coloration ou la décoloration des cheveux, de composition éventuellement à rincer à appliquer entre les deux étapes d'une permanente ou d'un défrisage, de composition à appliquer avant ou après une permanente, un défrisage, une coloration ou une décoloration des cheveux.

40. Composition selon l'une quelconque des revendications précédentes, **caractérisée en ce qu'**elle est se présente sous la forme d'un après-shampoing à rincer.

41. Procédé de traitement des matières kératiniques, telles que les cheveux, **caractérisé en ce qu'**il consiste à appliquer sur lesdites matières une quantité efficace d'une composition cosmétique selon l'une des revendications 1 à 39, puis à effectuer éventuellement un rinçage.
